(19) Europäisches Patentamt
European Patent Office
Office européen des brevets

(11) Numéro de publication : **0 119 899**
**B1**

(12) **FASCICULE DE BREVET EUROPÉEN**

(45) Date de publication du fascicule du brevet : **05.03.86**

(51) Int. Cl.[4] : **C 07 C 69/593**, C 07 C 67/343, C 07 C121/413, C 07 C120/00, C 07 C121/30

(21) Numéro de dépôt : **84400412.7**

(22) Date de dépôt : **01.03.84**

(54) **Nouveau procédé de préparation de dérivés de l'acide pen-4-énoïque.**

(30) Priorité : **01.03.83 FR 8303328**

(43) Date de publication de la demande :
**26.09.84 Bulletin 84/39**

(45) Mention de la délivrance du brevet :
**05.03.86 Bulletin 86/10**

(84) Etats contractants désignés :
**AT BE CH DE GB IT LI LU NL SE**

(56) Documents cités :
**FR-A- 2 341 553**
**US-A- 4 362 670**

(73) Titulaire : **ROUSSEL-UCLAF**
**35, boulevard des Invalides**
**F-75007 Paris (FR)**

(72) Inventeur : **Julia, Marc**
**57, rue Geoffroy St. Hilaire**
**F-75005-Paris (FR)**
Inventeur : **Cuvigny, Thérèse**
**3, Square de Port-Royal**
**F-75013-Paris (FR)**

(74) Mandataire : **Fritel, Hubert et al**
**ROUSSEL-UCLAF 111, route de Noisy Boîte Postale no. 9**
**F-93230 Romainville (FR)**

**EP 0 119 899 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un nouveau procédé de préparation de dérivés de l'acide pent-4-énoïque.

Des procédés consistant à faire réagir des composés de type malonique ou des analogues, c'est-à-dire des composés capables de fournir facilement des anions, avec des dérivés réactifs, dans le but de créer des liaisons carbone-carbone ont été décrits.

On connaît ainsi, par exemple, le procédé décrit dans le brevet français 2 341 553, consistant à condenser un malonate ou un cyanacétate d'alcoyle, en présence d'une base, avec un dérivé chloré d'un alcène substitué.

On peut encore citer le procédé décrit dans le brevet US-4 362 670, consistant à former des dérivés allylés de composés de type malonique ou analogue, en faisant réagir un carbonate d'allyle éventuellement substitué, avec un malonate d'alcoyle ou un analogue, sans la présence d'une base, mais en utilisant un catalyseur dérivé du palladium.

La présente invention a pour objet un procédé de préparation des composés de formule (I) :

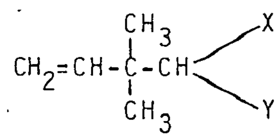

(I)

dans laquelle X représente un atome d'hydrogène ou un radical alcoxycarbonyl renfermant de 2 à 5 atomes de carbone ou un radical cyano et Y représente un atome d'hydrogène, un radical alcoxycarbonyl renfermant de 2 à 5 atomes de carbone, un radical cyano, un radical acyle renfermant de 2 à 7 atomes de carbone, ou un radical alkylsulfonyl renfermant de 1 à 4 atomes de carbone ou arylsulfonyl renfermant 6 ou 7 atomes de carbone, X et Y ne pouvant représenter en même temps un atome d'hydrogène, caractérisé en ce que l'on traite un composé de formule (II) :

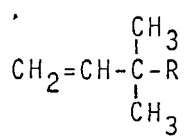

(II)

dans laquelle R représente un radical acyloxy renfermant de 1 à 7 atomes de carbone ou un radical alkylsulfonyl renfermant de 1 à 4 atomes de carbone ou arylsulfonyl renfermant 6 ou 7 atomes de carbone, par un anion dérivé d'un composé de formule (III) :

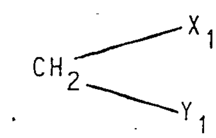

(III)

dans laquelle $X_1$ et $Y_1$ ont les valeurs de X et Y indiquées précédemment, à l'exception d'un atome d'hydrogène, en présence d'un complexe au palladium ou d'un complexe au nickel (o), et, le cas échéant, d'une quantité catalytique d'un ligand, pour obtenir un composé de formule ($I_A$) :

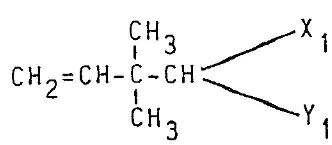

($I_A$)

dans laquelle $X_1$ et $Y_1$ sont définis comme précédemment, correspondant à un composé de formule (I), dans laquelle X et Y ont les définitions de $X_1$ et $Y_1$ précitées, composé de formule ($I_A$) que, le cas échéant, l'on soumet à une décarbalcoxylation, dans le cas où $X_1$ ou $Y_1$ représente un radical alcoxycarbonyl ou à une réduction dans le cas où $X_1$ représente un radical arylsulfonyl pour obtenir les composés de formule (I) correspondants.

Lorsque X ou Y représente un radical alcoxycarbonyl renfermant de 2 à 5 atomes de carbone, il s'agit de préférence d'un radical méthoxycarbonyl ou éthoxycarbonyl.

Lorsque Y représente un radical acyl renfermant de 2 à 7 atomes de carbone, il s'agit de préférence d'un radical acétyl, propionyl ou benzoyl.

Lorsque Y ou R représente un radical alkylsulfonyl, par alkyl, on entend de préférence, un radical méthyl ou éthyl.

Lorsque Y ou R représente un radical arylsulfonyl, il s'agit de préférence, d'un radical phénylsulfonyl ou d'un radical paratoluène sulfonyl.

Lorsque R représente un radical acyloxy, il s'agit de préférence d'un radical acétyloxy, propionyloxy, butyryloxy ou benzoyloxy.

La réaction du composé de formule (II) avec le composé de formule (III) est effectuée au sein d'un solvant organique qui est notamment un éther, plus particulièrement le tétrahydrofuranne ou l'éther éthylique, le diméthylsulfoxyde ou le diméthylformamide.

L'anion dérivé du composé de formule (III) est issu du dérivé obtenu par action d'un agent basique tel qu'un hydrure, un alcoolate alcalin ou un hydroxyde alcalin anhydre, sur ledit composé de formule (III). Cet anion est, de préférence, issu du dérivé sodé obtenu par action de l'hydrure de sodium.

L'invention a particulièrement pour objet un procédé tel que défini précédemment, caractérisé en ce que l'on utilise au départ un composé de formule (III) dans laquelle $Y_1$ représente un radical alcoxycarbonyl renfermant de 2 à 5 atomes de carbone ou un radical cyano, et plus particulièrement un procédé tel que défini précédemment, caractérisé en ce que l'on utilise au départ un composé de formule (III) dans laquelle $X_1$ représente un radical cyano.

L'invention a notamment pour objet un procédé tel que défini précédemment, caractérisé en ce que l'on utilise un complexe au palladium (II) et plus particulièrement un procédé tel que défini précédemment, caractérisé en ce que le complexe au palladium (II) est le bis ($\eta_3$-allyl) di-$\mu$-chlorodipalladium (II).

L'invention a aussi notamment pour objet un procédé tel que défini précédemment, caractérisé en ce que l'on utilise un complexe au palladium (o), et plus particulièrement un procédé tel que défini précédemment, caractérisé en ce que le complexe au palladium (o) est le tétrakis (triphénylphosphine) palladium ou le di(1,2-bis diphénylphosphinoéthane) palladium.

L'invention a aussi notamment pour objet un procédé tel que défini précédemment, caractérisé en ce que l'on utilise un complexe au nickel (o). Dans ce cas, le complexe au nickel (o) est de préférence préparé in situ par réduction d'un complexe au nickel (II), lequel est plus particulièrement un complexe formé par le dichlorure de nickel avec un composé formé par le dichlorure de nickel avec un composé choisi dans le groupe constitué par la triphényl phosphine, la tributyl phosphine, la triisopropyl phosphine, le diphényl phosphinoéthane, le diphényl phosphinopropane, le diphényl phosphinobutane, le diphényl phosphinohexane.

L'agent de réduction du complexe au nickel (II) est de préférence le chlorure d'isopropyl magnésium.

Dans des conditions préférentielles d'exécution du procédé de l'invention, lorsque l'on utilise un complexe au palladium, on opère en présence d'un ligand, lequel est de préférence choisi dans le groupe constitué par la triphényl phosphine, le diphényl phosphinoéthane, l'hexaméthylphosphotriamide, le phosphite de triéthyle, le phosphite de triphényle, l'hydroquinone.

La décarbalcoxylation du composé de formule ($I_A$) est effectuée par chauffage au sein d'un solvant organique, de préférence le diméthylsulfoxyde, en présence d'eau et, le cas échéant, d'un sel alcalin, qui peut être le chlorure de sodium, l'iodure de potassium ou le cyanure de sodium ou encore, d'une base organique.

La réduction du composé de formule ($I_A$) est effectuée par action d'un amalgame métallique tel que l'amalgame de sodium ou d'aluminium, la réaction étant effectuée au sein d'un alcanol inférieur tel que le méthanol ou l'éthanol.

Les dérivés de l'acide pent-4-énoïque de formule (I) sont des intermédiaires utiles pour la préparation de dérivés d'acides 2,2-diméthyl cyclopropane carboxyliques substitués par un groupement dihalovinyl, lesquels dérivés sont eux-mêmes des intermédiaires bien connus pour la préparation d'esters possédant des propriétés pesticides et notamment insecticides et acaricides.

La cyclisation des dérivés de formule (I) en dérivés d'acides 2,2-diméthyl cyclopropane carboxyliques a par exemple été décrite dans les brevets français 2 301 510, 2 318 143, 2 318 144, 2 351 943 ainsi que dans le brevet belge 847 865.

Les dérivés de formule (I) sont en général décrits dans les brevets ci-dessus.

Le procédé de l'invention présente par rapport aux procédés connus menant aux dérivés de formule (I), l'avantage de conduire en 2 stades au maximum à ces dérivés, avec des rendements remarquables et en utilisant au départ des matières premières simples d'accès et peu coûteuses. Il présente en outre l'avantage, essentiellement dans le cas de l'utilisation des composés de formule (III) dans laquelle $X_1$ représente un radical cyano et $Y_1$ un radical alcoxycarbonyl, de conduire très régio sélectivement à l'isomère souhaité, c'est-à-dire de conduire à la substitution du carbone le plus substitué de la molécule.

Les exemples suivants illustrent l'invention sans toutefois la limiter.

## Exemple 1

2-cyano 3,3-dimethyl pent-4-èn-1-oate d'éthyle. (utilisation d'un complexe au palladium).

On mélange sous gaz inerte pendant 10 minutes à température ambiante 9,2 mg de bis($\eta_3$-allyl) di-$\mu$-chloro dipalladium (II) et 26,2 mg de triphényl phosphine avec 3 $cm_3$ de tétrahydrofuranne.

On ajoute ensuite une solution de 512 mg de 3-acétoxy 3-méthyl but-1-ène dans 3 $cm_3$ de tétrahydrofuranne, puis une suspension de cyanacétate d'éthyle sodé (préparée à partir de 791 mg de cyanacétate d'éthyle et 262 mg d'une dispersion d'hydrure de sodium dans l'huile à 55 %, dans 18 $cm_3$ de tétrahydrofuranne). On maintient sous agitation à température ambiante et sous gaz inerte pendant 72 heures, ajoute de l'eau, extrait à l'éther éthylique, décante, sèche la phase organique et évapore le solvant. On chromatographie le résidu sur silice en éluant au mélange éther de pétrole-éther éthylique et isole 713 mg de produit attendu. ($Eb_{20}$ : 122-126 °C). Le produit renferme 93 % d'isomère attendu et 7 % d'isomère 2-cyano 5-méthyl hex-4-èn-1-oate d'éthyle.

## Exemple 2

3,3-diméthyl pent-4-ène nitrile.

On chauffe à 160 °C pendant 3 heures un mélange de 2,7 g de produit obtenu comme à l'exemple 1, 0,3 g de chlorure de sodium, 0,6 g d'eau et 10 $cm_3$ de diméthylsulfoxyde. On refroidit, reprend à l'eau, extrait à l'éther éthylique, sèche la phase organique et évapore le solvant. On obtient 1,34 g de produit attendu ($Eb_{20}$ : 58 °C).

## Exemple 3

2-cyano 3,3-diméthyl pent-4-èn-1-oate d'éthyle. (utilisation d'un complexe au nickel).

On mélange à température ambiante pendant 10 minutes 131 mg de dichloro (bis triphényl phosphine) nickel (II), 1,5 $cm_3$ de tétrahydrofuranne et 372 µl d'une solution 1,08 N de chlorure d'isopropyl magnésium dans l'éther éthylique.

On introduit ensuite une solution de 128 mg de 3-acétoxy 3-méthyl but-1-ène dans 2 $cm_3$ de tétrahydrofuranne puis une suspension de cyanacétate d'éthyle sodé (préparée à partir de 248 mg de cyanacétate d'éthyle et 88 mg d'une dispersion d'hydrure de sodium dans l'huile à 55 %, dans 6 $cm_3$ de tétrahydrofuranne). On maintient sous agitation à température ambiante pendant 48 heures.

On ajoute une solution de chlorure d'ammonium, extrait à l'éther éthylique, décante, sèche la phase organique et évapore le solvant. On chromatographie le résidu sur silice en éluant au mélange éther de pétrole-éther éthylique et obtient 126 mg de produit attendu, identique à celui obtenu à l'exemple 1. Le produit renferme 95 % d'isomère attendu et 5 % d'isomère 2-cyano 5-méthyl hex-4-èn-1-oate d'éthyle.

## Exemple 4 à 33

En opérant de manière analogue à celle décrite à l'exemple 1 ou 3, les exemples suivants ont été réalisés au départ du 3-acétoxy 3-méthyl but-1-ène (composé A) ou du 3-p-tolylsulfonyl 3-méthyl but-1-ène (composé B).

(Voir tableau pages suivantes)

| Exemple | Comp. II | Composé III | Catalyseur (note 1) (% molaire) | ligand (note 2) (% molaire) | Solvant | Température | Temps de réaction | Rendement | % d'isomère I obtenu |
|---|---|---|---|---|---|---|---|---|---|
| 4 | A | $CH_2(COOEt)_2$ | $Pd(Tpp)_4$ (5) | TPP (20) | Tétrahydro-furanne | Reflux | 36 h. | 68 % | 73 % |
| 5 | " | $CH_2(COOMe)_2$ | " | " | " | " | " | 80 % | 80 % |
| 6 | " | 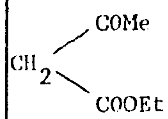 | " | " | " | " | " | 88 % | 55 % |
| 7 | " | 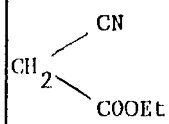 | " | " | " | " | " | 100 % | 86 % |
| 8 | " | 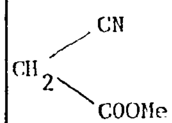 | $Pd(TPP)_4$ (2,5) | TPP (8) | " | " | " | 79 % | 92 % |
| 9 | " | 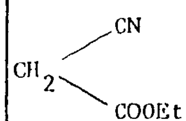 | $(\eta_3-C_3H_5)_2Pd_2Cl_2$ (2,5) | $P(OEt)_3$ (10) | " | " | " | 66 % | 70 % |
| 10 | " | " | $Pd(DPPE)_2$ (5) | DPPE (5) | " | " | " | 78 % | 73 % |
| 11 | " | 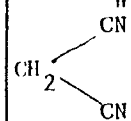 | $Pd(TPP)_4$ (5) | TPP (10) | " | " | " | 98 % | 95 % |
| 12 | " | " | $Pd(DPPE)_2$ (5) | DPPE (5) | " | " | " | 96 % | 79 % |
| 13 | " | 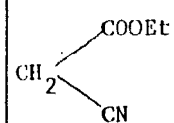 | $(\eta^3-C_3H_5)_2Pd_2Cl_2$ (5) | TPP (5) HQ (10) | " | 20°C | 48 h. | 90 % | 89 % |

| Exemple | Comp II | Composé III | Catalyseur (note 1) (% molaire) | Ligand (note 2) (% molaire) | Solvant | Température | Temps de réaction | Rendement | % d'isomère I obtenu |
|---|---|---|---|---|---|---|---|---|---|
| 14 | A | 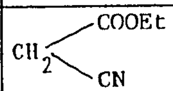 | $(\eta_3C_3H_5)_2Pd_2Cl_2$(5) | TPP (10) | Tétrahydro-furanne | 45°C | 36 h. | 90 % | 88 % |
| 15 | " | " | " | TPP (5) | " | 20°C | 72 h. | 82 % | 94 % |
| 16 | " | " | " (1,25) | TPP (2,5) | " | " | " | 98,5 % | 93 % |
| 17 | B | 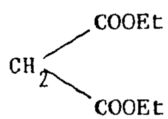 | $Pd(TPP)_4$ (5) | TPP (20) | " | Reflux | 36 h. | 88 % | 77 % |
| 18 | " | " | " | HMPT (50) | " | " | " | 100 % | 74 % |
| 19 | " | " | $(\eta^3C_3H_5)_2Pd_2Cl_2$(2,5) | TPP (15) | " | " | " | 89 % | 72 % |
| 20 | " | 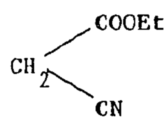 | $Pd(TPP)_4$ (5) | TPP (20) | " | " | " | 93 % | 89 % |

| Exemple | Comp. II | Composé III | Catalyseur (note 1) (% molaire) | Réducteur | Solvant | Température | temps de réaction | Rendement | % d'isomère I obtenu |
|---|---|---|---|---|---|---|---|---|---|
| 21 | A | 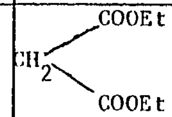 | $NiCl_2(TPP)_2$ (20) | $iP_rMgCl$ | THF | 20°C | 36 h. | 41 % | 65 % |
| 22 | " | 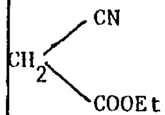 | " | " | " | " | 48 h. | 70 % | 95 % |
| 23 | " | " | $NiCl_2$(DPPB) (5) | " | " | 45°C | 24 h. | 89 % | 80 % |
| 24 | " | " | " | " | " | 35°C | 36 h. | 48 % | 90 % |
| 25 | " | " | " | " | DMSO/THF(5/3) | 45°C | " | 69 % | 95 % |
| 26 | " | " | $NiCl_2$(TPP) (20) | Zn | THF | " | " | 58 % | 96 % |
| 27 | " | 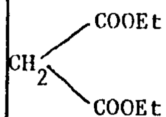 | $NiCl_2$(TBP) (5) | $iP_rMgCl$ | THF | 65°C | " | 30 % | 65 % |
| 28 | " | " | $NiCl_2$(DPPH) (5) | " | " | " | " | 55 % | 71 % |
| 29 | " | 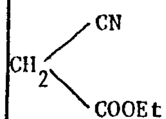 | $NiCl_2$(TBP) (20) | " | " | Reflux | 24 h. | 68 % | 82 % |
| 30 | " | " | $NiCl_2(TiP_2P)$ (20) | " | " | " | " | 34 % | 95 % |
| 31 | " | " | $NiCl_2$(DPPP) (5) | " | " | " | " | 80 % | 60 % |
| 32 | " | " | $NiCl_2$(DPPH) (5) | " | " | " | " | 83 % | 59 % |
| 33 | B | " | $NiCl_2$(DPPE) (5) | " | " | " | " | 68 % | 52 % |

— (1) (2)
TPP = triphényl phosphine ;
DPPB = diphényl phosphinobutane ;
TBP = tributyl phosphine ;
DPPH = diphényl phosphinohexane ;
$TiP_rP$ = triisopropyl phosphine ;
DPPP = diphényl phosphinopropane ;
DPPE = diphényl phosphinoéthane ;
HQ = hydroquinone ;
HMPT = hexaméthylphosphotriamide.

— Les pourcentages molaires indiqués s'entendent par rapport au composé A ou au composé B. Dans le cas d'un catalyseur au Pd (II), ils sont exprimés en atomes de Pd par rapport aux moles de A ou B.

**Revendications**

1. Procédé de préparation des composés de formule (I) :

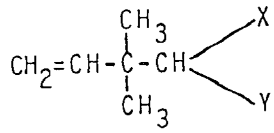

(I)

dans laquelle X représente un atome d'hydrogène ou un radical alcoxycarbonyl renfermant de 2 à 5 atomes de carbone ou un radical cyano et Y représente un atome d'hydrogène, un radical alcoxycarbonyl renfermant de 2 à 5 atomes de carbone, un radical cyano, un radical acyle renfermant de 2 à 7 atomes de carbone ou un radical alkylsulfonyl renfermant de 1 à 4 atomes de carbone ou arylsulfonyl renfermant 6 ou 7 atomes de carbone, X et Y ne pouvant pas représenter en même temps un atome d'hydrogène, caractérisé en ce que l'on traite un composé de formule (II) :

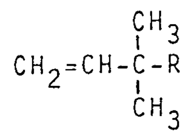

(II)

dans laquelle R représente un radical acyloxy renfermant de 1 à 7 atomes de carbone ou un radical alkylsulfonyl renfermant de 1 à 4 atomes de carbone ou arylsulfonyl renfermant 6 ou 7 atomes de carbone, par un anion dérivé d'un composé de formule (III) :

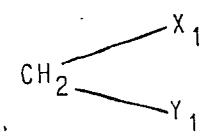

(III)

dans laquelle $X_1$ et $Y_1$ ont les valeurs de X et Y indiquées précédemment, à l'exception d'un atome d'hydrogène, en présence d'un complexe au palladium ou d'un complexe au nickel (o), et, le cas échéant, d'une quantité catalytique d'un ligand, pour obtenir un composé de formule ($I_A$) :

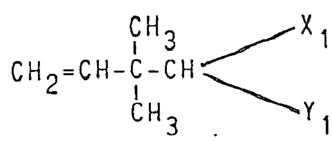

($I_A$)

dans laquelle $X_1$ et $Y_1$ sont définis comme précédemment, correspondant à un composé de formule (I), dans laquelle X et Y ont les définitions de $X_1$ et $Y_1$ précitées, composé de formule ($I_A$) que, le cas échéant, l'on soumet à une décarbalcoxylation, dans le cas où $X_1$ et $Y_1$ représente un radical alcoxycarbonyl, ou à une réduction, dans le cas où $X_1$ représente un radical arylsulfonyl pour obtenir les composés de formule (I) correspondants.

2. Procédé selon la revendication 1, caractérisé en ce que l'on utilise au départ un composé de formule (III) dans laquelle $Y_1$ représente un radical alcoxy carbonyl renfermant de 2 à 5 atomes de carbone ou un radical cyano.

3. Procédé selon la revendication 2, caractérisé en ce que l'on utilise au départ un composé de formule (III) dans laquelle $X_1$ représente un radical cyano.

4. Procédé selon l'une quelconque des revendications 1 à 3, caractérisé en ce que l'on utilise un complexe au palladium (II).

5. Procédé selon la revendication 4, caractérisé en ce que le complexe au palladium (II) est le bis ($\eta_3$-allyl) di-$\mu$-chloro-dipalladium (II).

6. Procédé selon l'une quelconque des revendications 1 à 3, caractérisé en ce que l'on utilise un complexe au palladium (o).

7. Procédé selon la revendication 6, caractérisé en ce que le complexe au palladium (o) est le tétrakis (triphényl phosphine) palladium ou le di(1,2-bis diphényl phosphino éthane) palladium.

8. Procédé selon l'une quelconque des revendications 1 à 3, caractérisé en ce que l'on utilise un complexe au nickel (o).

9. Procédé selon la revendication 8, caractérisé en ce que le complexe au nickel (o) est préparé in situ par réduction d'un complexe au nickel (II).

10. Procédé selon la revendication 9, caractérisé en ce que l'agent de réduction est le chlorure d'isopropyl magnésium.

11. Procédé selon la revendication 9, caractérisé en ce que le complexe au nickel (II) est un complexe formé par le dichlorure de nickel avec un composé choisi dans le groupe constitué par la triphényl phosphine, la tributyl phosphine, la triisopropyl phosphine, le diphényl phosphinoéthane, le diphényl-phosphino propane, le diphényl phosphinobutane, le diphényl phosphinohexane.

12. Procédé selon l'une quelconque des revendications 1 à 7, caractérisé en ce que le ligand est choisi dans le groupe constitué par la triphényl phosphine, le diphénylphosphinoéthane, l'hexaméthyl phosphotriamide, le phosphite de triéthyle, le phosphite de triphényle, l'hydroquinone.

**Claims**

1. Preparation process for compounds with the formula (I) :

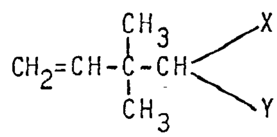

(I)

in which X represents a hydrogen atom or an alkoxycarbonyl radical containing from 2 to 5 carbon atoms or a cyano radical and Y represents a hydrogen atom, an alkoxycarbonyl radical containing from 2 to 5 carbon atoms, a cyano radical, an acyl radical containing from 2 to 7 carbon atoms or an alkylsulphonyl radical containing from 1 to 4 carbon atoms or an arylsulphonyl radical containing 6 or 7 carbon atoms, X and Y not being able to represent at the same time a hydrogen atom, characterized in that a compound with the formula (II) :

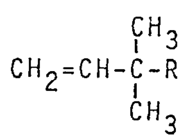

(II)

in which R represents an alkoxy radical containing from 1 to 7 carbon atoms or an alkylsulphonyl radical containing from 1 to 4 carbon atoms or an arylsulphonyl radical containing 6 or 7 carbon atoms, is treated with an anion derived from a compound with the formula (III) :

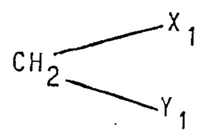

(III)

in which $X_1$ and $Y_1$ have the values of X and Y indicated previously, with the exception of a hydrogen atom, in the presence of a palladium complex or a nickel (o) complex, and, if the case arises, of a catalytic quantity of a ligand, so as to obtain a compound with the formula ($I_A$) :

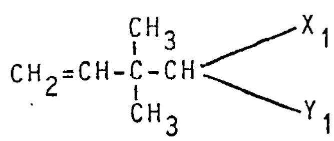

($I_A$)

in which $X_1$ and $Y_1$ are defined as previously, corresponding to a compound with the formula (I) in which X and Y have the previously cited definitions of $X_1$ and $Y_1$, which compound with the formula ($I_A$), if the case arises, is submitted to a decarbalkoxylation, in the case where $X_1$ and $Y_1$ represent an alkoxycarbonyl radical, or to a reduction, in the case where $X_1$ represents an arylsulphonyl radical so as to obtain the corresponding compounds with the formula (I).

2. Process according to claim 1, characterized in that at the start a compound with the formula (III) is used in which $Y_1$ represents an alkoxy carbonyl radical containing from 2 to 5 carbon atoms or a cyano radical.

3. Process according to claim 2, characterized in that at the start a compound with the formula (III) is used in which $X_1$ represents a cyano radical.

4. Process according to any one of the claims 1 to 3, characterized in that a palladium (II) complex is used.

5. Process according to claim 4, characterized in that the palladium (II) complex is bis ($\eta^3$-allyl) di-$\mu$-chloro-dipalladium (II).

6. Process according to any one of the claims 1 to 3, characterized in that a palladium (o) complex is used.

7. Process according to claim 6, characterized in that the palladium (o) complex is tetrakis (triphenyl phosphine) palladium or di(1,2-bis diphenyl phosphino ethane) palladium.

8. Process according to any one of the claims 1 to 3, characterized in that a nickel (o) complex is used.

9. Process according to claim 8, characterized in that the nickel (o) complex is prepared in situ by reduction of a nickel (II) complex.

10. Process according to claim 9, characterized in that the reducing agent is isopropyl magnesium chloride.

11. Process according to claim 9, characterized in that the nickel (II) complex is a complex formed by the nickel dichloride with a compound chosen from the group composed of triphenyl phosphine, tributyl phosphine, triisopropyl phosphine, diphenyl phosphinoethane, diphenylphosphino propane, diphenyl phosphinobutane, diphenyl phosphinohexane.

12. Process according to any one of the claims 1 to 7, characterized in that the ligand is chosen from the group composed of triphenyl phosphine, diphenylphosphinoethane, hexamethyl phosphotriamide, triethyl phosphite, triphenyl phosphite, hydroquinone.

**Patentansprüche**

1. Verfahren zur Herstellung der Verbindungen der Formel (I)

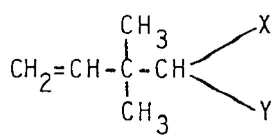

(I)

worin X ein Wasserstoffatom oder einen Alkoxycarbonylrest mit 2 bis 5 Kohlenstoffatomen oder einen Cyanorest bedeutet und Y ein Wasserstoffatom, einen Alkoxycarbonylrest mit 2 bis 5 Kohlenstoffatomen, einen Cyanorest, einen Acylrest mit 2 bis 7 Kohlenstoffatomen oder einen Alkylsulfonylrest mit 1 bis 4 Kohlenstoffatomen oder Arylsulfonylrest mit 6 oder 7 Kohlenstoffatomen bedeutet, wobei X und Y nicht gleichzeitig ein Wasserstoffatom bedeuten können, dadurch gekennzeichnet, daß man eine Verbindung der Formel (II)

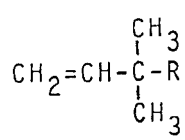

(II)

worin R einen Acyloxyrest mit 1 bis 7 Kohlenstoffatomen oder einen Alkylsulfonylrest mit 1 bis 4 Kohlenstoffatomen oder Arylsulfonylrest mit 6 oder 7 Kohlenstoffatomen bedeutet, mit einem Anion, das sich von einer Verbindung der Formel (III)

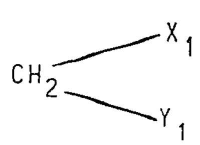

(III)

worin $X_1$ und $Y_1$ die vorstehend für X und Y angegebenen Bedeutungen mit Ausnahme eines Wasserstoffatoms besitzen, ableitet, in Gegenwart eines Palladium-Komplexes oder eines Nickel (O)-Komplexes und gegebenenfalls eines katalytischen Menge eines Liganden behandelt, um eine Verbindung der Formel ($I_A$)

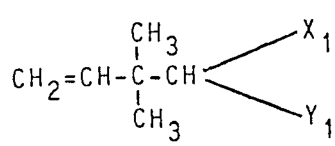

($I_A$)

worin $X_1$ und $Y_1$ die vorstehende Bedeutung besitzen, entsprechend einer Verbindung der Formel (I), worin X und Y die vorstehenden Bedeutungen von $X_1$ und $Y_1$ besitzen, zu erhalten, die Verbindung der Formel ($I_A$) gegebenenfalls, wenn $X_1$ und $Y_1$ einen Alkoxycarbonylrest bedeutet, einer Decarbalkoxylierung unterzieht oder, wenn $X_1$ einen Arylsulfonylrest bedeutet, einer Reduktion unterzieht, um die entsprechenden Verbindungen der Formel (I) zu erhalten.

2. Verfahren gemäß Anspruch 1, dadurch gekennzeichnet, daß man von einer Verbindung der Formel (III) ausgeht, worin $Y_1$ einen Alkoxycarbonylrest mit 2 bis 5 Kohlenstoffatomen oder einen Cyanorest bedeutet.

3. Verfahren gemäß Anspruch 2, dadurch gekennzeichnet, daß man von einer Verbindung der Formel (III) ausgeht, worin $X_1$ einen Cyanorest bedeutet.

4. Verfahren gemäß einem der Ansprüche 1 bis 3, dadurch gekennzeichnet, daß man einem Palladium (II)-Komplex verwendet.

5. Verfahren gemäß Anspruch 4, dadurch gekennzeichnet, daß der Palladium (II)-Komplex Bis-($\eta_3$-allyl-di-$\mu$-chlor-dipalladium (II) ist.

6. Verfahren gemäß einem der Ansprüche 1 bis 3, dadurch gekennzeichnet, daß man einen Palladium (O)-Komplex verwendet.

7. Verfahren gemäß Anspruch 6, dadurch gekennzeichnet, daß der Palladium (O)-Komplex Tetrakis-(triphenylphosphin)-palladium oder Di-(1,2-bis-diphenyl-phosphinoethan)-palladium ist.

8. Verfahren gemäß einem der Ansprüche 1 bis 3, dadurch gekennzeichnet, daß man einen Nickel (O)-Komplex verwendet.

9. Verfahren gemäß Anspruch 8, dadurch gekennzeichnet, daß der Nickel (O)-Komplex in situ durch Reduktion eines Nickel (II)-Komplexes hergestellt wird.

10. Verfahren gemäß Anspruch 9, dadurch gekennzeichnet, daß das Reduktionsmittel Isopropylmagnesiumchlorid ist.

11. Verfahren gemäß Anspruch 9, dadurch gekennzeichnet, daß der Nickel (II)-Komplex ein Komplex ist, der aus Nickeldichlorid und einer Verbindung, ausgewählt unter Triphenylphosphin, Tributylphosphin, Triisopropylphosphin, Diphenylphosphinoethan, Diphenylphosphinopropan, Diphenylphosphinobutan und Diphenylphosphinohexan, gebildet wird.

12. Verfahren gemäß einem der Ansprüche 1 bis 7, dadurch gekennzeichnet, daß der Ligand unter Triphenylphosphin, Diphenylphosphinoethan, Hexamethyl-phosphortrisamid, Triethylphosphit, Triphenylphosphit und Hydrochinon ausgewählt wird.